(19) Europäisches Patentamt
European Patent Office
Office européen des brevets

(11) **EP 0 715 526 B1**

(12) **EUROPÄISCHE PATENTSCHRIFT**

(45) Veröffentlichungstag und Bekanntmachung des Hinweises auf die Patenterteilung:
**05.11.2003 Patentblatt 2003/45**

(21) Anmeldenummer: **94926215.8**

(22) Anmeldetag: **20.08.1994**

(51) Int Cl.⁷: **A61L 15/22**, A61L 15/42

(86) Internationale Anmeldenummer:
**PCT/EP94/02782**

(87) Internationale Veröffentlichungsnummer:
**WO 95/005857 (02.03.1995 Gazette 1995/10)**

(54) **WUNDVERSORGUNGSARTIKEL MIT SELEKTIVEM ABSORPTIONSVERMÖGEN**

WOUND TREATING ARTICLE WITH SELECTIVE ABSORPTION POWER

ARTICLE DE SOINS DE PLAIES A CAPACITE D'ABSORPTION SELECTIVE

(84) Benannte Vertragsstaaten:
**DE ES FR GB IT NL**

(30) Priorität: **23.08.1993 DE 4328190**

(43) Veröffentlichungstag der Anmeldung:
**12.06.1996 Patentblatt 1996/24**

(73) Patentinhaber: **Beiersdorf Aktiengesellschaft
20245 Hamburg (DE)**

(72) Erfinder:
• **ACHTERBERG, Volker
  D-20257 Hamburg (DE)**
• **KENNDOFF, Jochen-Werner
  D-21149 Hamburg (DE)**
• **WELLING, Cecilia
  D-21465 Reinbeck (DE)**

(56) Entgegenhaltungen:
**EP-A- 0 147 588      EP-A- 0 163 150
EP-A- 0 190 814      WO-A-92/17518
DE-A- 4 233 289**

**Beschreibung**

Die vorliegende Erfindung betrifft Wundauflagen oder Wundversorgungsartikel gemäß Anspruch 1, die in der Lage sind, durch Wasserentzug eine Konzentrationserhöhung der körpereigenen Proteine auf der Wundoberfläche zu bewirken und deren Verwendung.

Bekannte Wundversorgungsartikel auf Basis natürlicher oder synthetischer Materialien sind so konzipiert, daß sie austretende Wundflüssigkeit absorbieren. Dabei wird das Sekret in seiner Gesamtheit gebunden und damit von der Wundoberfläche entfernt.

Der Nachteil davon ist, daß auch Sekret-Bestandteile, die vom Organismus speziell für die Wundheilung herantransportiert werden, mit aufgesaugt und entfernt werden.

Ziel war es, solche Materialien zu synthetisieren und bereitzustellen, die selektiv das Wasser absorbieren und dadurch die Proteinanteile auf der Oberfläche der Wunde erhöhen.

Überraschenderweise sind dafür natürliche oder künstliche Polymermaterialien geeignet, die wasserabsorbierende natürliche oder synthetische Materialien enthalten.

Gegenstand der Erfindung sind daher Wundauflagen oder Wundversorgungsartikel gemäß Anspruch 1, die ein selektives Absorptionsverhalten aufweisen und natürliche oder künstliche Polymermaterialien und wasserabsorbierende natürliche oder synthetische Materialien enthalten.

Selektives Absorptionsverhalten bedeutet hier, daß die Wundauflagen oder Wundversorgungsartikel, die z. B. solche Wundauflagen besitzen, beim Kontakt mit der Wunde nur Wasser oder im wesentlichen nur Wasser (gegebenenfalls mit gelöstem Salz) aus der Wunde oder Wundflüssigkeit aufnehmen und daß sich die sonstigen in der Wundflüssigkeit verbleibenden Bestandteile darin anreichern bzw. konzentriert werden. Insbesondere werden körpereigene Proteine und Wachstumsfaktoren wie z.B. PDGF auf der Wundoberfläche angereichert.

Hintergrund der Erfindung ist, daß insbesondere Wundversorgungsprodukte, die bei schwer heilenden Wunden zum Einsatz kommen, die Wundheilung passiv unterstützen sollten. Eine solche Eigenschaft ist allerdings auch bei allen anderen Wundheilungsprozessen wünschenswert und daher nicht auf die Gruppe der schwer heilenden Wunden beschränkt.

Eine passive Unterstützung kann sein, daß körpereigene Proteine, die eine Wundheilung beschleunigen und vom Körper zu diesem Zwecke im Wundbereich vermehrt auftreten, auf der Wundoberfläche gehalten werden. Wichtig ist hierbei, daß dabei ein feuchtes Wundmillieu erhalten bleibt, so daß die Proteine in ihrer Funktion nicht wesentlich beeinträchtigt werden.

Gegenstand der Erfindung ist auch die Verwendung der erfindungsgemäßen Wundabdeckungsmaterialien zum Anreichern von körpereigenen Proteinen in der Wundflüssigkeit und das Verfahren zum Anreichern von körpereigenen Proteinen in der Wundflüssigkeit.

Weiterhin ist die Verwendung der erfindungsgemäß geeigneten Materialien zur Herstellung von Wundabdeckungen oder Wundversorgungsartikeln mit selektivem Absorptionsverhalten Gegenstand der Erfindung.

Verwendet werden Wundauflagen bzw. Polymermaterialien auf Polyurethanbasis, insbesondere Polyurethangele oder geschäumte Polyurethangele, d.h. Polyurethanschaumgele bzw. Polyurethangelschäume, mit jeweils den wasserabsorbierenden Materialien.

Besonders geeignet als erfindungsgemäße Wundauflagen sind Polyurethangele, wie sie aus EP-A-0 057 839 und EP-A-0 147 588 bekannt sind, die flüssigkeitsabsorbierende Materialien als Füllstoff enthalten. Dabei können die Gele in geschäumter oder in ungeschäumter Form vorliegen.

Weiterhin sind Polyisobutylen-Polymere geeignet, die mit wasserabsorbierenden Materialien gefüllt sind.

Wundauflagen auf Basis solcher Polymeren sind bekannt. Sie enthalten Polyisobutylen, Polyterpenharz und Fettsäureester. Geeignete wasserabsorbierende Materialien sind z.B. Carboxymethylcellulose und Karaya-Pulver. Auch andere erfindungsgemäße wasserabsorbierende Materialien sind geeignet.

Die Wundauflagen oder Wundversorgungsartikel sind erhältich aus

1. einem Polyurethangel, welches

(A) 15-62 Gew.-%, vorzugsweise 20-57 Gew.-%, besonders bevorzugt 25-47 Gew.-%, bezogen auf die Summe aus (A) und (B), eines kovalent vernetzten Polyurethans als hochmolekulare Matrix und

(B) 85-38 Gew.-%, vorzugsweise 80-43 Gew.-%, besonders bevorzugt 75-53 Gew.-%, bezogen auf die Summe aus (A) und (B) einer oder mehrerer in der Matrix durch Nebenvalenzkräfte fest gebundenen Polyhydroxylverbindungen mit einem mittleren Molekulargewicht zwischen 1000 und 12000, vorzugsweise zwischen 1500 und 8000, besonders bevorzugt zwischen 2000 und 6000, und einer mittleren OH-Zahl zwischen 20 und 112, vorzugsweise zwischen 25 und 84, besonders bevorzugt zwischen 28 und 56, als flüssigem Dispersionsmittel, wobei das Dispersionsmittel im wesentlichen frei ist an Hydroxylverbindungen mit einem Molekulargewicht unter 800, vorzugsweise unter 1000, besonders bevorzugt unter 1500, sowie gegebenenfalls

(C) 0-100 Gew.-%, bezogen auf die Summe aus (A) und (B), an Füll- und/oder Zusatzstoffen enthält, und welches erhältlich ist durch Umsetzung einer Mischung von

a) einem oder mehreren Polyisocyanaten,
b) einer oder mehreren Polyhydroxylverbindungen mit einem mittleren Molekulargewicht zwischen 1000 und 12000, und einer mittleren OH-Zahl zwischen 20 und 112,
c) gegebenenfalls Katalysatoren oder Beschleunigern für
die Reaktion zwischen Isocyanat- und Hydroxylgruppen
sowie gegebenenfalls
d) aus der Polyurethanchemie an sich bekannten Füll und Zusatzstoffen,

wobei diese Mischung im wesentlichen frei ist von Hydroxylverbindungen mit einem Molekulargewicht unter 800, die mittlere Funktionalität der Polyisocyanate ($F_I$) zwischen 2 und 4 liegt, die mittlere Funktionalität der Polyhydroxylverbindung ($F_P$) zwischen 3 und 6 beträgt und die Isocyanatkennzahl (K) der Formel

$$K = \frac{300 \pm X}{(F_I \cdot F_P) - 1} + 7$$

gehorcht, in welcher $X \leq 120$, vorzugsweise $X \leq 100$, besonders bevorzugt $X \leq 90$ ist und die Kennzahl K bei Werten zwischen 15 und 70 liegt, wobei die angegebenen Mittelwerte von Molekulargewicht und OH-Zahl als Zahlenmittel zu verstehen sind, und

2. einem Wasser absorbierendem Material,

wobei diese Mischungen gegebenenfalls mit einem Schäumungsmittel geschäumt sein können.

Es werden nichtwäßrige Schäumungsmittel verwendet,

Hyodrophile Schäume zur Wundversorgung werden in WO 92/17518 beschrieben.

Die erfindungsgemäßen Polyurethangele sind in der EP-Patentschrift 147 588 beschrieben und können aus den an sich aus der Polyurethanchemie bekannten Ausgangsverbindungen nach an sich bekannten Verfahren hergestellt werden, wie sie z.B. in DE-OS 31 03 499 und DE-OS 31 03 500 beschrieben werden. Wesentlich ist jedoch, daß bei der Auswahl der gelbildenden Komponenten die oben definierten Bedingungen eingehalten werden, da sonst anstelle von selbsthaftenden Gelen klebfreie, elastische Gele erhalten werden.

Erfindungsgemäß bevorzugte Polyhydroxylverbindungen sind Polyetherpolyole, wie sie in den oben genannten Deutschen Offenlegungsschriften ausführlich genannt sind.

Als Polyisocyanatkomponente sind sowohl (cyclo)aliphatische als auch aromatische Isocyanate geeignet. Bevorzugte (cyclo)aliphatische Polyisocyanate sind 1,6-Hexamethylen-diisocyanat sowie dessen Biurete und Trimerisate bzw. hydrierte Diphenylmethandiisocyanat ("MDI")-Typen. Bevorzugte aromatischen Polyisocyanate sind solche, die durch Destillation erhalten werden, wie MDI-Gemische aus 4,4'- und 2,4'-Isomeren oder 4,4'-MDI, sowie Toluylendiisocyanat ("TDI")-Typen. Die (cyclo)aliphatischen und die aromatischen Isocyanate können aufgrund von Modifizierungen, z.B. Biuretisierung, Trimerisierung oder Präpolymerisierung, auch höherfunktionelle Anteile beinhalten.

Die Diisocyanate können insbesondere z.B. aus der Gruppe der unmodifizierten aromatischen oder aliphatischen Diisocyanate oder aber aus durch Präpolymerisierung mit Aminen, Polyolen oder Polyetherpolyolen gebildeten modifizierten Produkten gewählt werden. Bevorzugt sind 4,4'-Diisocyanatodiphenylmethan, oder aber aus durch Präpolymerisierung mit Polyolen oder Polyetherpolyolen gebildete modifizierte Produkte. Als sehr günstig hat sich beispielsweise durch Präpolymerisierung mit Tripropylenglycol verflüssigtes 4,4'-Diisocyanatodiphenylmethan erwiesen.

Bevorzugt werden ein oder mehrere Polyetherpolyole verwendet, die durch Anlagerung von Propylenoxid und gegebenenfalls Ethylenoxid an übliche Startermoleküle erhältlich sind und wie sie z.B. in EP 147 588 beschrieben sind.

Bevorzugte Polyether-Polyole sind in der nachfolgenden Tabelle zusammengestellt. Sie wurden durch Anlagerung von Propylenoxid und gegebenenfalls Ethylenoxid an die angegebenen Startermoleküle in an sich bekannter Weise hergestellt.

| Polyol Nr. Funktio-nalität | Propylenoxid | Ethylenoxid | Startermolekül | OH-Zahl | OH- |
|---|---|---|---|---|---|
| | % | % | | | |
| 1 | 80 | 20 | PET | 36 | 4 |
| 2 | 100 | - | Sorbit | 46 | 6 |
| 3 | 73 | 27 | Sorbit | 30 | 6 |
| 4 | 45 | 55 | TMP | 56 | 3 |
| 5 | 100 | - | PET | 72 | 4 |
| 6 | 100 | - | TMP | 56 | 3 |
| 7 | 90 | 10 | Sorbit | 83 | 6 |
| 8 | 100 | - | EDA | 61 | 4 |
| 9 | 83 | 17 | TMP | 35 | 3 |
| 10 | 100 | - | PET | 45 | 4 |
| PET = Pentaerythrit TMP = Trimethylolpropan EDA = Ethylendiamin | | | | | |

Die Startermoleküle für die Polyetherpolyole sind z.B. Pentaerythrit, Sorbit, Trimethylolpropan oder Ethylendiamin.

Besonders vorteilhaft werden die Katalysatoren oder Beschleuniger gewählt aus der Gruppe bestehend aus

- Organischen Säuren, insbesondere

    p-Toluolsulfonsäure,
    n-Butylphosphorsäure

- Organozinnverbindungen, einschließlich deren Salze

    organischer und anorganischer Säuren, insbesondere
    Zinnnaphthenat, Zinnbenzoat, Dibutylzinndioctoat,
    Dibutylzinndiacetat, Zinn-II-ethylhexoat und
    Dibutylzinndiacetat

- Eisensalzen höherer Fettsäuren, insbesondere Eisenstearat
- Aminen, zum Beispiel Isophorondiamin, Methylendianilin, Imidazole
- tertiären Aminen, insbesondere Trialkylamine,

wobei die Alkylreste jeweils vorteilhaft 2 - 6 Kohlenstoffatome haben.

Erfindungsgemäß werden die Ausgangskomponenten so gewählt, daß im gelbildenden Reaktionsgemisch die mittlere NCO-Funktionalität zwischen 2 und 4, die mittlere Polyol-Funktionalität zwischen 3 und 6 und die Isocyanatkennzahl zwischen 15 und 70, vorzugsweise zwischen 18 und 55, besonders bevorzugt zwischen 20 und 45, liegen.

Die Polyurethan-Gele können gegebenenfalls aus der Polyurethan-Chemie an sich bekannte Zusatzstoffe enthalten, wie z.B. Füllstoffe und Kurzfasern auf anorganischer oder organischer Basis, Metallpigmente, oberflächenaktive Substanzen oder flüssige Streckmittel wie Substanzen mit einem Siedepunkt von über 150°C.

Als organische Füllstoffe seien beispielsweise Schwerspat, Kreide, Gips, Kieserit, Soda, Titandioxid, Ceroxid, Quarzsand, Kaolin, Ruß und Mikrohohlkugeln genannt.

An organischen Füllstoffen können z.B. Pulver auf Basis von Polystyrol, Polyvinylchlorid, Harnstoff-Formaldehyd und Polyhydrazodicarbonamid eingesetzt werden. Als Kurzfasern kommen z.B. Glasfasern von 0,1 - 1 mm Länge oder Fasern organischer Herkunft, wie z.B. Polyester- oder Polyamidfasern, in Frage. Metallpulver, wie z.B. Eisen- oder Kupferpulver, können ebenfalls bei der Gelbildung mitverwendet werden. Um den erfindungsgemäßen Gelen die gewünschte Färbung zu verleihen, können die bei der Einfärbung von Polyurethanen an sich bekannten Farbstoffe oder Farbpigmente auf organischer oder anorganischer Basis verwendet werden, wie z.B. Eisenoxid- oder Chromoxidpigmente, Pigmente auf Phthalocyanin- oder Monoazo-Basis. Als oberflächenaktive Substanzen seien z.B. Cellulosepulver, Aktivkohle, und Kieselsäurepräparate genannt.

Als flüssige Streckmittel können beispielsweise Ethylstearat, Laurinsäurehexylester, Isopropoylmyristat, Isopropylpalmitat oder Dodecylsulfonsäureester verwendet werden.

Weiterhin können als flüssige Streckmittel auch höhermolekulare Polyole eingesetzt werden, deren Hydroxylgruppen verethert, verestert oder urethanisiert sind, sowie Paraffinöle und Silikonöle.

Der Gehalt an Füllstoffen und Streckmitteln in dem Gel kann vorzugsweise bis zu 50 Gew.-%, bezogen auf das Gesamtgewicht des Gels, betragen.

Zur Modifizierung der Hafteigenschaften der Gele können gegebenenfalls Zusätze von polymeren Vinylverbindungen, Polyacrylaten und sonstigen in der Klebstoff-Technik üblichen Copolymeren bzw. auch Klebemittel auf Naturstoffbasis bis zu einem Gehalt von 10 Gew.-%, bezogen auf das Gewicht der Gelmasse, zugegeben werden.

Die Füll- und Zusatzstoffe können auch aus den üblichen Substanzklassen gewählt werden. Insbesondere sind vorteilhaft Farbstoffe, Pigmente Lichtschutzmittel, Konservierungsmittel, Duftstoffe, antimikrobiell wirksame Substanzen, sonstige Wirkstoffe wie beispielsweise kühlend wirkende Substanzen (z.B. Menthol) oder solche, die die Durchblutung fördern oder ein Wärmegefühl erzeugen.

Bevorzugte Wasser absorbierende Materialien sind als Superabsorber bekannte Wasser absorbierende Salze von Polyacrylaten und deren Copolymeren, insbesondere die Natrium- oder Kaliumsalze. Sie können unvernetzt oder vernetzt sein und sind auch als Handelsprodukte erhältlich. Insbesondere sind solche Produkte geeignet, wie sie in der DE-A-37 13 601 offenbart werden und auch Superabsorber der neuen Generation mit nur noch geringen Anteilen an austrockenbarem Wasser, wie sie in der Literatur beschrieben sind.

Bevorzugte Produkte sind schwach vernetzte Polymerisate auf der Basis Acrylsäure/Natriumacrylat. Solche Natriumpolyacrylate sind als Favor 922-SK (Chemische Fabrik Stockhausen GmbH, Deutschland) erhältlich.

Weitere Absorber, z.B. Carboxymethylcellulose und Karaya sind ebenfalls geeignet.

Die Gewichtsmenge des Wasser absorbierenden Materials kann bis zum 1,5-fachen der Gewichtsmenge der Polyhydroxyverbindung betragen. Bevorzugt beträgt die Gewichtsmenge des Wasser absorbierenden Materials 100 bis 5 Gew.-%, vorteilhaft auch 20 bis 70 Gew.-%, insbesondere 35 bis 60 Gew.-%, bezogen auf das Gewicht der Polyhydroxylverbindung.

Das Wasser absorbierende Material liegt vorzugsweise in fein gemahlener Form vor, insbesondere wenn dünne Schaumschichten gewünscht werden. Die Teilchengröße der Wasser absorbierenden Materialien, d.h. der Durchmesser des überwiegenden Anteils der Teilchen dieses Materials liegt vorzugsweise unter 300 μm, bevorzugt unter 200 μm, insbesondere aber unter 100μm. Bevorzugt beträgt die Teilchengröße 1 bis 100 μm, insbesondere 1 bis 30 um.

Als Schäumungsmittel können nichtwäßrige Schäumungsmittel dienen.

Als nichtwäßriges Schäumungsmittel können wasserfreie , vorzugsweise inerte Gase oder niedrig siedende Lösungsmittel verwendet werden, die sich dann in den Polyurethangelschäumen in fein verteilter Form befinden. Es können auch Gemische dieser Schäumungsmittel verwendet werden. Besonders bevorzugt werden Luft, Stickstoff oder Kohlendioxid oder Gemische dieser Gase, insbesondere aber Stickstoff. Eine zusätzliche Schäumung durch einen Wasserzusatz ist nicht erforderlich. Geeignete Lösungsmittel sind niedrigsiedende Lösungsmittel wie Ester, Ketone, Alkane und chlorierte Kohlenwasserstoffe, z.B. Alkylacetate wie Ethylacetat oder Trichlorfluormethan, Dichlordifluormethan, Methylenchlorid sowie die Treibgas-Ersatzstoffe.

Der Schäumungsgrad läßt sich durch die eingearbeiteten Mengen an Schäumungsmittel in weiten Grenzen variieren. So läßt sich die Dichte des Schaumes auf Werte von etwa 0,15 - 1,1 g/cm$^3$ einstellen. Dieser Dichtebereich ist für die Wundbehandlung geeignet.

Alle verwendeten Ausgangsstoffe sind im werentlichen wasserfrei . Geeignet sind aber auch wasserhaltige Stoffe, in denen das Wasser so gebunden ist, daß es nicht reagiert.

| Vorteilhaft sind die erfindungsgemäßen Polyurethankomponenten erhältlich aus | |
|---|---|
| 20 - 95 | Gew.-% Polyhydroxylverbindung |
| 1 - 60 | Gew.-% Polyisocyanat |
| 5 - 60 | Gew.-% Superabsorber |
| 0.0001 - 10 | Gew.-% Beschleuniger |

und gegebenenfalls einem nichtwäßrigen Schäumungsmittel in einer Menge, die Dichten von 0,15 bis 1,1 g/cm$^3$ ergibt.

| Bevorzugt sind die erfindungsgemäßen Polyurethankomponenten erhältlich aus | |
|---|---|
| 50 - 70 | Gew.-% Polyhydroxylverbindung |
| 2 - 25 | Gew.-% Polyisocyanat |
| 5 - 40 | Gew.-% Superabsorber |

(fortgesetzt)

| Bevorzugt sind die erfindungsgemäßen Polyurethankomponenten erhältlich aus | |
|---|---|
| 0.001 - 1 | Gew.-% Beschleuniger |

und gegebenenfalls einem nichtwäßrigen Schäumungsmittel in einer Menge, die Dichten von 0,3 bis 1,0 g/cm$^3$ ergibt.

Vorteilhaft ist es, Gewichts-Verhältnisse von Polyhydroxylverbindung : Polyisocyanat von (5 - 35) : 1 zu wählen, insbesondere etwa (10 - 25) : 1. Die Topfzeit als Maß für die Verarbeitbarkeit dieser Massen liegt zwischen 0.5 und 30 Minuten.

Die eingesetzten Polyetherpolyole können beispielsweise Handelsprodukte sein wie Levagel VP SN 100 und als Diisocyanate haben sich Handelsprodukte wie Desmodur PF, Desmodur N 100, IPDI und Desmodur W als geeignet erwiesen (Handelsprodukte der Bayer AG).

Für die Beschleuniger, so das Dibutylzinndilaurat, ist Desmorapid Z/SN, für Zinn-II-ethylhexoat Desmorapid SO ein vorteilhaftes Handelsprodukt (Bayer AG).

Gegenstand der Erfindung ist auch das Verfahren zur Herstellung der erfindungsgemäßen Wundauflagen, das dadurch gekennzeichnet ist, daß man

1. die vorstehend unter 1. genannten und definierten Komponenten (A), (B) und (C) sowie a), b), c), und d) des Polyurethangeles

2. ein Wasser absorbierendes Material und gegebenenfalls

3. ein nichtwäßriges Schäumungsmittel,

vereinigt und miteinander mischt und gegebenenfalls schäumt, wobei Gase während des Vermischens eingebracht, insbesondere eingerührt oder eingeschlagen werden und eingebrachte Lösungsmittel durch Verdampfen in der Masse die Schäumung bewirken, wozu die Masse gegebenenfalls erwärmt wird.

Die Erwärmung erfolgt vorzugsweise in dem sich gegebenenfalls anschließenden Härtungsprozeß, insbesondere bei Temperaturen von 20° bis 140°C und Zeiten von 24 Stunden bis zu einigen Sekunden.

Die Herstellung der Gele kann insbesondere gemäß EP 147 588 auf verschiedene Weise erfolgen.

Man kann z.B. nach dem one-shot- oder dem Prepolymer-Verfahren arbeiten. Beim one-shot Verfahren werden alle Komponenten, d.h. Polyole, Di- und/oder Polyisocyanate, die Isocyanat-Polyadditionsreaktion beschleunigende Katalysatoren und gegebenenfalls Füll- und Zusatzstoffe und die übrigen Komponenten auf einmal zusammengeben und intensiv miteinander vermischt.

Beim Prepolymer-Verfahren sind zwei Arbeitsweisen möglich, entweder stellt man zunächst ein Isocyanat-Prepolymer her, indem man einen entsprechenden Anteil der Polyolmenge mit der gesamten, für die Gelbildung vorgesehenen Isocyanatmenge umsetzt, und fügt dann dem erhaltenen Prepolymer die restliche Menge an Polyol sowie gegebenenfalls Füll -und Zusatzstoffe und die übrigen Komponenten zu und mischt intensiv. Oder man setzt die gesamte, für die Gelbildung vorgesehene Menge an Polyol mit einem Teil der Isocyanatmenge zu einem Hydroxyl-Prepolymer um und mischt anschließend die restliche Menge an Isocyanat zu.

Eine erfindungsgemäß besonders vorteilhafte Arbeitsweise ist eine Variante aus dem one-shot-Verfahren und dem Hydroxyl-Prepoylmer-Verfahren. Hierbei werden das Polyol bzw. Polyolgemisch, gegebenenfalls die Füll- und Zusatzstoffe und die übrigen Komponenten der Katalysator und zwei verschiedene Diisocyanate in einem Schuß zusammengegeben und intensiv vermischt, wobei ein Diisocyanat aliphatischer Natur ist. Man kann davon ausgehen, daß durch die stark unterschiedliche Reaktivität der beiden Diisocyanate zunächst ein Hydroxylprepolymer entsteht, das sodann innerhalb von Minuten mit dem anderen Diisocyanat unter Gelbildung reagiert.

Bei diesen Verfahrensweisen kann die Förderung, Dosierung und Mischung und gegebenenfalls Schäumung der Einzelkomponenten und der Übrigen Komponenten oder Komponentengemische mit den für den Fachmann in der Polyurethan-Chemie an sich bekannten Vorrichtungen erfolgen.

Das Schäumen mit einem nichtwäßrigen Schäumungsmittel vorzugsweise durch Einrühren oder Einschlagen von Luft ist an sich bekannt, bietet aber hier im speziellen besondere Vorteile:

Die erfindungsgemäßen, vorteilhaften Polyurethangele können leicht zwischen gering und sehr stark klebend durch Variationen des Verhältnisses von NCO/OH gesteuert werden. Diese exakte Steuerung ist in einfacher Weise nur dann möglich, wenn im wesentlichen wasserfrei gearbeitet wird. Der Grund hierfür liegt in der extrem hohen Wasserabsorptionsfähigkeit der Superabsorber, da zugesetztes Wasser, das für den Schäumungsprozeß zur Verfügung stehen muß, in einer unkontrollierten Weise dem Prozeß entzogen werden kann und wird, so daß es ungleich schwieriger ist, die Produkteigenschaften gezielt einzustellen.

Die erfindungsgemäßen Wundauflagen erfüllen alle gestellten Anforderungen.

Die erfindungsgemäßen Wundauflagematerialien können nach einer vorteilhaft etwa 0,5 - 30 Minuten messenden Topfzeit zu flächigen Gebilden ausgegossen oder ausgestrichen werden. Auf diese Weise sind Dicken von 0,015 mm bis 15 cm problemlos erreichbar. Durch den Einsatz von fein gemahlenem Absorber sind beim Verstreichen dünne Masseaufträge bis zu 15 g / m² gut herstellbar. Es ist aber auch möglich und vorteilhaft, Gegenstände aus den erfindungsgemäßen Materialien anzufertigen, welche nicht flächig, sondern ausgeprägt raumfüllend sind, beispielsweise durch übliche Gußverfahren.

Die entstehenden Schäume sind offenporige Schäume. Es ist also nicht notwendig, sie durch Schnittverfahren in solche umzuwandeln.

Vorteilhaft können die erfindungsgemäßen Wundauflage-Materialien nach an sich bekannten Verfahren auch auf flächige Träger, z.B. Gewebe, Gewirke, Vliese oder Folien aufgetragen werden, die ebenfalls Gegenstand der Erfindung sind.

Die in diesen erfindungsgemäßen selbsthaftenden Flächengebilden enthaltenen Trägermaterialien können unterschiedlichster Herkunft sein, d.h. Materialien auf Basis von natürlichen, halb- oder vollsynthetischen Rohstoffen sowie organischen oder anorganischen Ursprungs sind verwendbar. Beispielsweise lassen sich Kunststoff- und Metallfolien, Matten, Vliese, Gewirke, Gestricke oder Gewebe aus organischem oder anorganischem Fasermaterial, Papier und Schaumstoff-Folien oder auch Kombinationen aus diesen Trägermaterialien einsetzen. Für die medizinische Anwendung bevorzugt sind luft- und feuchtigkeitsdurchlässige Flächengebilde, z.B. mikro- und makroporöse Kunststoffolien und Vliese, sowie elastische textile Trägermaterialien, insbesondere Stretchgewebe, und Mullbinden.

Gegenstand der Erfindung ist ferner ein Verfahren zur Herstellung selbsthaftender Flächengebilde auf Basis von mit erfindungsgemäßen Materialien beschichteten Trägermaterialien; das Verfahren ist dadurch gekennzeichnet, daß man die oben definierten Materialien oder zur Schaumgelbildung befähigten Reaktionsgemische auf die Oberfläche eines Trägermaterials aufbringt, z.B. nach Direktverfahren oder Umkehrverfahren durch Gießen oder Rakeln, wobei die Oberfläche mit dem gelbildenden Reaktionsgemisch gegebenenfalls nur teilweise bedeckt wird. Die Schichtdicke kann z.B. zwischen 0,015 mm und 150 mm, vorzugsweise zwischen 0,1 mm und 50 mm, besonders bevorzugt zwischen 0,1 mm und 6 mm, liegen.

Die Herstellung der erfindungsgemäßen Flächengebilde kann kontinuierlich oder diskontinuierlich erfolgen. Die Arbeitsweise hängt von den vorgegebenen, mit einer Haftschicht zu versehenden Flächengebilden ab. Wenn man bereits zugeschnittene Trägermaterialien vorliegen hat, ist oftmals eine diskontinuierliche Arbeitsweise vorteilhaft. Die kontinuierliche Arbeitsweise empfiehlt sich bei der Beschichtung von Trägermaterialien, die in endloser Form, z.B. als Rollenware, vorliegen. Der Auftrag der Schaum-Haftschicht auf das Trägermaterial kann dabei direkt oder nach dem Umkehrverfahren erfolgen. Das Reaktionsgemisch läßt sich bei den genannten Verfahren auch, bevor es durch die Reaktion erstarrt, rakeln.

Obwohl die Kohäsion der erfindungsgemäßen Wundauflagen gut ist, kann eine Nachbehandlung der erfindungsgemäßen Gele und Schäume und Flächengebilde durch die Bestrahlung mit gamma-Strahlen, wodurch die Kohäsion in der Gelschicht verbessert wird, vorteilhaft sein.

Obwohl es, wie erwähnt, möglich ist, die Hafteigenschaften zu steuern, so daß die Wundauflagen selbstklebend sein können, können sie vorteilhaft auf einer der beiden oder auch beiden Großflächen mit einer üblichen Selbstklebemasse beschichtet werden.

Gegenstand der Erfindung ist auch die Verwendung der erfindungsgemäßen Wundauflagen oder der damit hergestellten selbsthaftenden Flächengebilde in der Medizin, z.B. zur Behandlung von Defektwunden bzw. zu deren Prophylaxe, insbesondere als Heftpflaster oder Wundverband oder Wundpflaster, Bandage oder Binde.

Die Wundauflage, vorzugsweise die vorabbeschriebenen flächigen Gebilde, vorteilhaft aber auch die beschichteten Träger, können beispielsweise dazu dienen, Oberflächenwunden oder chirurgische Wunden zu versorgen.

Ein besonders vorteilhaftes Anwendungsgebiet ist die Versorgung tiefer Wunden. Insbesondere deshalb, weil die erfindungsgemäßen Schäume aufeinander haften können. Eine oder mehrere dünne Schaumschichten, die einzeln sehr bequem in geeignete Formen zu schneiden sind, können z.B. modelliert und der Wunde angepaßt werden, ohne daß es weiterer Fixierungshilfsmittel bedürfte. Dadurch wird die gleiche Wirkung erzielt wie mit dicken Schaumschichten.

Die erfindungsgemäßen Wundauflagen wirken in überraschender Weise selektiv und reichern Wundheilungsrelevante Proteine, wie z.B. Wachstums faktoren (z.B. PDGF, EGF oder basisches FGF), Immunglobuline, Albumin oder Fibrinogen in der Wundflüssigkeit an. Die Konzentrationen dieser Proteine kann vorzugsweise mindestens auf das 1,5- bis 1,9-fache der normalen Konzentration ansteigen.

Die erfindungsgemäßen Wundauflagen bzw. Wundversorgungsartikel und ihre Verwendung fördern daher die Wundheilung und sind für schwer und schlecht heilende Wunden besonders geeignet und können insbesondere für großflächige und tiefe Wunden verwendet werden.

Im Rahmen der vorliegenden Anmeldung sind, soweit nicht anders angegeben, alle Mengen und Prozentangaben auf das Gewicht und die Gesamtzusammensetzung der Zubereitung bezogen.

Die nachfolgenden Beispiele sollen die Erfindung erläutern, ohne daß aber beabsichtigt ist, die Erfindung auf diese Beispiele zu beschränken.

Beispiele

Die folgenden Reaktionskomponenten werden in einem kommerziell verfügbaren Schaummischkopf mit kommerziell verfügbaren Dosiereinrichtungen zusammengeführt und in diesem innig vermischt. Dann wird die Masse auf ein silikonisiertes Papier gestrichen und mit einem weiteren silikonisierten Papier eingedeckt. Anschließend läßt man die Masse bei 80°C ausreagieren. Zur Untersuchung der Eigenschaften werden beide Trennpapiere entfernt.

Beispiel 1

| Polyetherpolyol (Levagel VP SN 100) | 100 kg |
|---|---|
| Superabsorber (Favor SAB 922-SK) | 39 kg |
| Dibutylzinndilaurat | 0.01 kg |
| Diisocyanat (Desmodur PF) | 6.6 kg |
| Stickstoff | 300 1 |

Es resultiert ein schwach klebender, offenporiger Schaum mit 0,45 g/cm$^3$ Dichte. Das Material kann nach 90 Minuten etwa 25 g Wasser pro Gramm seines Eigengewichts aufnehmen.

Beispiel 2

| Polyetherpolyol (Levagel VP SN 100) | 100 kg |
|---|---|
| Superabsorber (Favor SAB 922-SK) | 39 kg |
| Dibutylzinndilaurat | 0.01 kg |
| Diisocyanat (Desmodur PF) | 6.6 kg |
| Stickstoff | 270 1 |

Es resultiert ein schwach klebender, offenporiger Schaum mit 0,55 g/cm$^3$ Dichte. Das Material kann nach 90 Minuten etwa 10 g Wasser pro Gramm seines Eigengewichts aufnehmen.

Beispiel 3

| Polyetherpolyol (Levagel VP SN 100) | 100 kg |
|---|---|
| Superabsorber (Favor SAB 922-SK) | 54 kg |
| Dibutylzinndilaurat | 0.08 kg |
| Diisocyanat (Desmodur PF) | 6.6 kg |
| Stickstoff | 300 1 |

Es resultiert ein klebender, offenporiger Schaum mit einer Dichte von 0,53 g/cm$^3$. Das Material hat nach 90 Minuten 115 g Wasser pro Gramm Eigengewicht aufgenommen.

Die Produkte dieser und der folgenden Beispiele können auch einseitig mit einer nicht oder nur schwer ablösbaren, flexiblen Folie aus Polyester oder Polyurethan kaschiert werden, wobei das Material dann etwa 40% seiner Quellfähigkeit (nach 90 Minuten) verliert.

Die Schichtdicke beträgt etwa 2 mm.

Werden in den vorstehenden Beispielen erfindungsgemäß statt Stickstoff andere Gase wie Luft oder Gasgemische oder Kohlendioxid verwendet, so erhält man gleiche Ergebnisse.

Beispiel 4

| Polyetherpolyol (Levagel VP SN 100) | 100 kg |
|---|---|
| Superabsorber (Favor SAB 922-SK) | 39 kg |
| Dibutylzinndilaurat | 0.01 kg |
| Diisocyanat (Desmodur PF) | 6.6 kg |
| Stickstoff, so daß eine Dichte von 0,45 g / cm$^3$ erhalten wird. | |

Hierzu sind etwa 235 - 285 1 Stickstoff geeignet. Schwankungen treten auf, da herstellungsbedingt Teilmengen des Stickstoffs ausgasen können.

Es resultiert ein schwach klebender, auf sich selbst haftender, offenporiger Schaum. Das Material kann bei 3 mm Dicke in 90 Minuten etwa 70 g Wasser pro Gramm seines Eigengewichts aufnehmen.

Beispiel 5

| Polyetherpolyol (Levagel VP SN 100) | 100 kg |
|---|---|
| Superabsorber (Favor SAB 922-SK) | 39 kg |
| Dibutylzinndilaurat | 0.01 kg |
| Diisocyanat (Desmodur PF) | 6.6 kg |
| Stickstoff, so daß eine Dichte von 0,5 g/cm$^3$ erhalten wird. | |

Hierzu sind etwa 200 - 250 1 Stickstoff geeignet. Schwankungen treten auf, da herstellungsbedingt Teilmengen des Stickstoffs ausgasen können.

Es resultiert ein schwach klebender, auf sich selbst haftender, offenporiger Schaum. Das Material kann nach 90 Minuten etwa 40 g Wasser pro Gramm seines Eigengewichts aufnehmen.

Beispiel 6

| Polyetherpolyol (Levagel VP SN 100) | 100 kg |
|---|---|
| Superabsorber (Favor SAB 922-SK) | 39 kg |
| Dibutylzinndilaurat | 0.03 kg |
| Diisocyanat (Desmodur PF) | 5,7 kg |
| Stickstoff, so daß eine Dichte von 0,54 g/cm$^3$ erreicht wird. | |

Hierzu sind etwa 165-215 1 Stickstoff geeignet. Schwankungen treten auf, da herstellungsbedingt Teilmengen des Stickstoffs ausgasen können.

Es resultiert ein schwach klebender, gut auf sich selbst haftender offenporiger Schaum. Die Klebkraft auf Stahl wurde mit etwa 0,4 N/cm bestimmt. Die Wasseraufnahme nach 90 Min. beträgt etwa 85 g Wasser/g Probe. Die Schaumdicke beträgt ca. 5mm.

Werden in den vorstehenden Beispielen erfindungsgemäß statt Stickstoff andere Gase wie Luft oder Gasgemische oder Kohlendioxid verwendet, so erhält man gleiche Ergebnisse.

Beispiele 7 - 9

Die folgenden Reaktionskomponenten werden in einem kommerziell verfügbaren Schaummischkopf mit kommerziell verfügbaren Dosiereinrichtungen zusammengeführt und in diesem innig vermischt. Dann wird die Masse auf ein silikonisiertes Papier gestrichen und mit einer Polyurethanfolie eingedeckt. Anschließend läßt man die Masse bei 80°C ausreagieren. Zur Untersuchung der Eigenschaften wird das Trennpapier entfernt.

Beispiel 7

| Polyetherpolyol (Levagel VP SN 100) | 100 kg |
|---|---|
| Superabsorber (Favor SAB 922-SK) | 38 kg |
| Dibutylzinndilaurat | 0.02 kg |
| Diisocyanat (Desmodur PF) | 5.7 kg |

Stickstoff, so daß eine Dichte von 0,52 g/cm$^3$ erhalten wird. Hierzu sind etwa 180 - 230 1 Stickstoff geeignet. Schwankungen treten auf, da herstellungsbedingt Teilmengen des Stickstoffs ausgasen können.

Es resultiert ein schwach klebender, offenporiger Schaum.

Das Material kann nach 90 Minuten etwa 35 g Wasser pro Gramm seines Eigengewichts aufnehmen. Die Klebkraft auf Stahl beträgt etwa 0,3 N/cm. Die Schichtdicke beträgt ca. 3 mm. Die Flüssigkeit wird im wesentlichen vertikal angesaugt und praktisch nicht zur Seite abgeführt.

Der Schaum kann zusätzlich fixiert werden. Die Wasserdampfdurchlässigkeit beträgt ca. 1/3 der ursprünglichen Wasserdampfdurchlässigkeit von rund 6500 g/m$^2$/24h der Polyurethanfolie. Die Foliendicke beträgt etwa 25 µm. Die Folie ist im Handel erhältlich (Rexham PU 77).

Beispiel 8

| Polyetherpolyol (Levagel VP SN 100) | 100 kg |
|---|---|
| Superabsorber (Favor SAB 922-SK) | 38 kg |
| Dibutylzinndilaurat | 0.02 kg |
| Diisocyanat (Desmodur PF) | 5.3 kg |

Stickstoff, so daß eine Dichte von 0,71 g/cm$^3$ erhalten wird. Hierzu sind etwa 80 - 130 1 Stickstoff geeignet. Schwankungen treten auf, da herstellungsbedingt Teilmengen des Stickstoffs ausgasen können.

Es resultiert ein auf Haut gut klebender, offenporiger Schaum. Das Material kann nach 90 Minuten etwa 17 g Wasser pro Gramm seines Eigengewichts aufnehmen. Die Klebkraft auf Stahl beträgt um 1,1 N/cm. Die Schichtdicke beträgt ca. 0,75 mm.

Die Schichtdicke der Polyurethanfolie (Rexham PU 77) liegt bei etwa 25 µm und weist eine Wasserdampfdurchlässigkeit von etwa 6500 g/m$^2$/24h auf. Sie reduziert sich durch Aufbringen der Masse um etwa 60%.

Beispiel 9

| Polyetherpolyol (Levagel VP SN 100) | 100 kg |
|---|---|
| Superabsorber (Favor SAB 922 SK) | 38 kg |
| Dibutylzinndilaurat | 0,02 kg |
| Diisocyanat (Desmodur PF) | 5,3 kg |

Stickstoff, so daß eine Dichte von 0,66 g/cm$^3$ erhalten wird. Hierzu sind etwa 100 bis 150 1 Stickstoff geeignet. Schwankungen treten auf, da herstellungsbedingt Teilmengen des Stickstoffs ausgasen können.

Man erhält einen sehr gut auf Haut klebenden Schaum.

Die Klebkraft auf Stahl wird zu etwa 0,6 N/cm bestimmt.

Die Wasseraufnahme beträgt ca. 13 g Wasser/g Probe. Es wurde eine Polyurethanfolie von 50 µm Dicke mit einer Wasserdampfdurchlässigkeit von 2100 g/m$^2$/24h verwendet (Rexham 91i). Die Schichtdicke beträgt 0,2 mm.

Werden in den vorstehenden Beispielen erfindungsgemäß statt Stickstoff andere Gase wie Luft oder Gasgemische oder Kohlendioxid verwendet, so erhält man entsprechende Ergebnisse.

Beispiel 10

Die Herstellung erfolgt mit Ausnahme der Schäumung entsprechend dem vorstehend angegebenen Verfahren. Es wurde kein Schäumungsmittel zugesetzt.

| Polyetherpolyol (Levagel VP SN 100) | 100 kg |
|---|---|
| Superabsorber (Favor SAB 922-SK) | 54 kg |
| Dibutylzinndilaurat | 0.08 kg |
| Diisocyanat (Desmodur PF) | 6.6 kg |

Es resultiert eine Wundauflage mit einer Dichte von 1,2 g/cm$^3$. Das Material hat nach 90 Minuten 8 g Wasser pro Gramm Eigengewicht aufgenommen.

Die Schichtdicke beträgt etwa 1,7 mm, die Klebkraft etwa 2,8 N/cm.

Die Produkte aller Beispiele können auch einseitig mit einer nicht oder nur schwer ablösbaren, flexiblen Folie aus Polyester kaschiert werden, wobei das Material dann etwa 40% seiner Quellfähigkeit (nach 90 Minuten) verliert.

Entsprechend den vorstehenden Beispielen erhaltene Wundauflagen wurden mit der auf der Wunde liegenden Seite nach oben weisend mit 10 ml Humanplasma, angereichert mit 100 ng/ml rek. PDGF-AB, in einer Filterkammer (4,5 cm φ) einen Tag lang bei Raumtemperatur inkubiert. Anschließend erfolgte eine Untersuchung der Zusammensetzung des im Überstand befindlichen Humanplasmas.

Der Gesamtprotein-Gehalt wurde nach der Bradford-Methode ermittelt, der Gehalt an Immunglobulinen, Albumin und Fibrinogen erfolgte turbidimetrisch mittels spezifischer Antigen-Antikörper-Kopplung. Der PDGF-Gehalt wurde mit Hilfe einer ELISA-Methode ermittelt.

Es resultierte ein signifikanter Anstieg der Konzentrationen der getesteten Proteine auf mindestens das 1,5- bis 1,9-fache der Ausgangswerte.

**Patentansprüche**

**1.** Verwendung eines, ein wasserabsorbierendes Material enthaltenen, geschäumten Polyurethangels zur Herstellung von Wundversorgungsartikeln zum Anreichern von körpereigenen Proteinen in der Wundflüssigkeit, wobei das Polyurethangel umfasst

(A) 15 - 62 Gew.%, bezogen auf die Summe aus (A) und (B), eines kovalent vernetzten Polyurethans als hochmolekulare Matrix,

(B) 38 - 85 Gew.%, bezogen auf die Summe aus (A) und (B), einer oder mehrerer in der Matrix durch Nebenvalenzkräfte fest gebundenen Polyhydroxylverbindungen mit einem mittleren Molekulargewicht zwischen 1000 und 12000 und einer mittleren OH-Zahl zwischen 20 und 112 als flüssigem Dispersionsmittel, wobei das Dispersionsmittel im wesentlichen frei an Hydroxylverbindungen mit einem Molekulargewicht unter 800 ist, und

(C) 0 - 100 Gew.%, bezogen auf die Summe aus (A) und (B), Füll- und/oder Zusatzstoffe, sowie ein Wasser absorbierendes Mittel,

wobei das Polyurethangel erhältlich ist durch Umsetzung einer Mischung von

a) einem oder mehrerer Polyisocyanaten,

b) einer oder mehrerer Polyhydroxylverbindungen entsprechend (B)

c) gegebenenfalls Katalysatoren oder Beschleunigern für die Reaktion zwischen Isocyanatund Hydroxylgruppen sowie

d) gegebenenfalls Füll- und/oder Zusatzstoffe,

wobei diese Mischung im wesentlichen frei an Hydroxylverbindungen mit einem Molekulargewicht unter 800 ist, die mittlere Funktionalität der Polyisocyanate (FI) zwischen 2 und 4 liegt, die mittlere Funktionalität der Polyhydroxylverbindung (FP) zwischen 3 und 6 liegt und die Isocyanatkennzahe (K) der Formel

$$K = \frac{300 \pm X}{(FI * FP) - 1} + 7$$

gehorcht, in welcher $X \leq 120$ und $15 < K < 70$,

**dadurch gekennzeichnet, dass**
das Polyurethangel mit einem nicht wässrigen Schäumungsmittel geschäumt ist und
das alle verwendeten Ausgangsstoffe im wesentlichen wasserfrei sind.

**2.** Verwendung gemäß Anspruch 1 zur Herstellung von Wundversorgungsartikeln zum Anreichern von Wachstumsfaktoren in der Wundflüssigkeit.

**Claims**

**1.** Use of a foamed polyurethane gel which contains a water-absorbing material for manufacturing wound care articles to enrich endogenous proteins in the wound fluid, the polyurethane gel comprising

(A) 15-62% by weight, based on the sum of (A) and (B), of a covalently crosslinked polyurethane as a high molecular weight matrix,
(B) 38-85% by weight, based on the sum of (A) and (B), of one or more polyhydroxyl compounds bound firmly in the matrix by secondary valency forces and having an average molecular weight of between 1 000 and 12 000, and an average OH number of between 20 and 112, as a liquid dispersion medium, the dispersion medium being essentially free of hydroxyl compounds having a molecular weight of below 800, and
(C) 0-100% by weight, based on the sum total of (A) and (B), of filler and/or additive materials, and also a water-absorbing agent,
the polyurethane gel being obtainable by reaction of a mixture of

a) one or more polyisocyanates,
b) one or more polyhydroxyl compounds conforming to (B)
c) if appropriate catalysts or accelerators for the reaction between isocyanate and hydroxyl groups and also
d) if appropriate filler and/or additive materials, this mixture being essentially free of hydroxyl compounds having a molecular weight of below 800, the average functionality of the polyisocyanates ($F_I$) being between 2 and 4, the average functionality of the polyhydroxyl compound ($F_P$) being between 3 and 6 and the isocyanate characteristic number (K) obeying the formula

$$K = \frac{300 \pm X}{(FI * FP) - 1} + 7$$

in which $X \leq 120$ and $15 < K < 70$,
**characterized in that**
the polyurethane gel has been foamed with a non-aqueous foaming agent and **in that** all the starting materials used are essentially anhydrous.

**2.** Use according to Claim 1 for producing wound care articles for enriching growth factors in the wound fluid.

**Revendications**

**1.** Utilisation d'un matériau absorbant l'eau, contenant un gel de polyuréthanne expansé, dans la fabrication d'articles de soin de la plaie, pour la concentration de protéines endogènes dans le liquide de la plaie, le gel de polyuréthanne comprenant

(A) 15-62 % en poids, par rapport à la somme de (A) et (B), d'un polyuréthanne réticulé par covalence, en tant que matrice de masse moléculaire élevée, et

(B) 38-85 % en poids, par rapport à la somme de (A) et (B), d'un ou plusieurs composés polyhydroxylés fixés solidement dans la matrice par des forces de valences secondaires, ayant une masse moléculaire moyenne comprise entre 1 000 et 12 000 et un indice moyen de groupes OH compris entre 20 et 112, en tant que dispersant liquide, le dispersant étant essentiellement exempt de composés hydroxylés ayant une masse moléculaire inférieure à 800, et

(C) 0-100 % en poids, par rapport à la somme de (A) et (B), d'additifs et/ou de charges,

12

ainsi qu'un agent absorbant l'eau, le gel de polyuréthanne pouvant être obtenu par réaction d'un mélange de

a) un ou plusieurs polyisocyanates,
b) un ou plusieurs composés polyhydroxylés selon (B),
c) éventuellement des catalyseurs ou des accélérateurs pour la réaction entre des groupes isocyanate et des groupes hydroxy, ainsi que
d) éventuellement des charges et additifs, ce mélange étant essentiellement exempt de composés hydroxylés ayant une masse moléculaire inférieure à 800, la fonctionnalité moyenne des polyisocyanates (FI) étant comprise entre 2 et 4, la fonctionnalité moyenne du composé polyhydroxylé (FP) étant comprise entre 3 et 6 et la constante d'isocyanate (K) obéissant à la formule

$$K = \frac{300 \pm X}{(F_I \cdot FP) - 1} + 7$$

dans laquelle $X \leq 120$ et $15 < K < 70$,
**caractérisé en ce que** le gel de polyuréthanne est expansé à l'aide d'un agent d'expansion non aqueux et **en ce que** tous les produits de départ sont essentiellement anhydres.

2. Utilisation selon la revendication 1, dans la fabrication d'articles de soin de la plaie, pour la concentration de facteurs de croissance dans le liquide de la plaie.